# EUROPÄISCHE PATENTSCHRIFT

(11) **EP 2 327 393 B1**
(45) Veröffentlichungstag und Bekanntmachung des Hinweises auf die Patenterteilung: **02.01.2019**
(21) Anmeldenummer: 10008114.0
(22) Anmeldetag: 04.08.2010
(51) Int. Cl.: A61K 8/35, A61Q 19/08

(54) **Verwendung von Zingeron gegen Altershaut**
Use of zingerone to combat skin ageing
Utilisation de zingeron pour les peaux matures

(30) Priorität: 27.11.2009 DE 102009055917
(43) Veröffentlichungstag der Anmeldung: 01.06.2011
(73) Patentinhaber: BEIERSDORF AG, 20245 Hamburg (DE)
(72) Erfinder: Heuser, Stefan, 90762 Fürth (DE); Winnefeld, Marc, 22880 Wedel (DE); Siegner, Ralf, 25421 Pinneberg (DE); Holtmann, Ursula, 22309 Hamburg (DE); Ahlheit, Sabrina, 22303 Hamburg (DE)
(74) Vertreter: Wilke, Jochen

(56) Entgegenhaltungen:
- EP-A1- 1 203 577
- EP-A1- 1 938 789
- CN-A- 1 289 835
- JP-A- 2003 104 876
- JP-A- 2008 208 132
- US-A1- 2002 051 800
- US-A1- 2007 259 057
- US-A1- 2009 220 625

## Beschreibung

Die vorliegende Erfindung betrifft die Verwendung von Zingeron zur Behandlung und Prophylaxe der Symptome der intrinsischen und/oder extrinsischen Hautalterung.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Insbesondere nimmt im Alter das subkutane Fettgewebe, insbesondere in der Peripherie (z.B. im Gesicht) ab, sodaß es zu typischen Altershauterscheinungen kommen kann.

Die vorliegende Erfindung betrifft insbesondere die Verwendung von Zingeron zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung die Verwendung von Zingeron in kosmetischen Zubereitungen, die imstande sind, das subkutane Fettgewebe der Altershaut zur gesteigerten Fettsynthese zu stimulieren.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Es hat sich überraschenderweise herausgestellt, daß Zingiberon die Differenzierung von Prä-Adipozyten zu Adipozyten und die Akkumulation von Triglyceriden in Adipozyten anregt.

Zingiberon (auch Zingeron, Vanillylaceton, 4-(4-Hydroxy-3-methoxyphenyl)butan-2-on) ist durch die folgende Struktur gekennzeichnet:

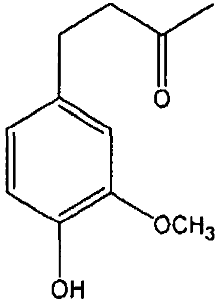

Zingeron ist ein Stoff, von dem fälschlicherweise häufig behauptet wird, daß er in Ingwer zu finden sei. Tatsächlich jedoch ist Zingeron eine Substanz, die erst durch den Abbau von in Ingwer vorkommenden Substanzen wie z.B. Gingerolen entsteht und deren Vorkommen in Extrakten ein Maß für deren mangelhafte und kaum nutzbare Qualität ist (Hänsel, Stichler; Pharmakognosie Phytopharmazie, 7. Auflage, Springer-Verlag, S. 617). In der Tat ist es so, daß wirtschaftlich und biologisch sinnvolle Mengen nur durch chemische Synthese gewonnen werden können.

Erfindungsgemäß ist daher die Verwendung von Zingiberon zur Behandlung und Prophylaxe der Symptome der intrinsischen und/oder extrinsischen Hautalterung.

Bevorzugt enthalten kosmetische oder dermatologische Zubereitungen 0,001 - 10 Gew.-%. besonders bevorzugt 0,05 - 5 Gew.-% Zingiberon, bezogen auf die Gesamtzusammensetzung der Zubereitungen.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Sofern die kosmetische oder dermatologische Zubereitung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen.

Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Die wäßrige Phase der Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure. Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältem versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propän, Butan, Isobutan) geeignet, die allein oder in Mischung miteinander eingesetzt werden können. Auch Druckluft ist vorteilhaft zu verwenden.

Die nachfolgenden Beispiele sollen die vorliegende Erfindung verdeutlichen.

### Anti-Falten-Creme (Beispiele 1 - 2)

| **INCI - Bezeichnung** | **Gew.-%** | **Gew.-%** |
|---|---|---|
| Stearinsäure | 2,50 | 2,50 |
| Glycerylstearat | 1,00 | 1,00 |
| C12-15 Alkylbenzoate | 5,00 | 5,00 |
| Caprylsäure/Caprinsäuretriglyceride | 2,50 | 2,50 |
| Cetylalcohol | 2,00 | 2,00 |
| Stearylalcohol | 2,00 | 2,00 |
| Cyclomethicon | 1,00 | 1,00 |
| Dicaprylylcarbonat | 2,00 | 2,00 |
| Dimethicon | 1,00 | 1,00 |
| Glycerin | 5,00 | 5,00 |
| Methylparaben | 0,20 | - |
| Phenoxyethanol | 0,40 | 0,10 |
| Propylparaben | 0,10 | 0,10 |
| Na-disulfit | - | 0,15 |
| Carbomer | 0,10 | 0,10 |
| Acrylate / C10-30 Alkylacrylatcrosspolymer | - | 0,30 |
| Trinatrium EDTA | 0,2 | 0,20 |
| Tapiokastärke | 1,50 | 1,50 |
| Zingeron | 0,15 | 0,5 |
| NaOH | q.s. | q.s. |
| Parfum | q.s. | q.s. |
| Wasser | ad 100 | ad 100 |

### Anti-Falten-Creme (Beispiele 3 - 4)

| **INCI - Bezeichnung** | **Gew.-%** | **Gew.-%** |
|---|---|---|
| Glycerylstearatcitrat | 2,00 | 3,00 |
| Behenylalcohol | 1,00 | - |
| Glycerylstearat | - | 1,00 |
| C12-15 Alkylbenzoate | 2,50 | 3,00 |
| Caprylsäure/Caprinsäuretriglyceride | 2,50 | 2,00 |
| Cetylalcohol | 2,00 | 2,00 |
| Cyclomethicon | 2,00 | 2,00 |
| Dicaprylylcarbonat | 2,50 | - |
| Dimethicon | 1,00 | - |
| Glycerin | 6,00 | 8,00 |
| Methylparaben | 0,05 | 0,20 |
| Phenoxyethanol | 0,20 | 0,40 |
| Propylparaben | 0,10 | 0,10 |
| Na-Disulfit | 0,10 | - |
| Carbomer | 0,10 | 0,20 |
| Natriumpolyacrylate | 0,30 | - |
| Xanthangummi | - | 0,15 |
| Talkum | 1,00 | 1,00 |
| Zingeron | 0,50 | 2,00 |
| Pimpinella anisum Extrakt | - | 1,00 |
| Butylmethoxydibenzoylmethan | - | 2,00 |
| Ethylhexylmethoxycinnam ate | - | 2,00 |
| Titandioxid | 0,50 | - |
| Octocrylen | 1,00 | 3,00 |
| NaOH | q.s. | q.s. |
| Parfum | q.s. | q.s. |
| Wasser | ad 100 | ad 100 |

### Anti-Falten-Creme (Beispiele 5 - 6)

| **INCI - Bezeichnung** | **Gew.-%** | **Gew.-%** |
|---|---|---|
| Polyglyceryl-3 Methylglucosdistearat | 2,50 | 2,50 |
| Sorbitanstearat | 1,00 | 3,00 |
| C12-15 Alkylbenzoate | 2,50 | 2,50 |
| Caprylsäure/Caprinsäuretriglyceride | 2,50 | 2,50 |
| Stearylalcohol | 1,00 | 1,50 |
| Cyclomethicon | 3,00 | 1,00 |
| Dicaprylylcarbonat | 2,50 | - |
| Paraffinum Liquidum | - | 1,00 |
| Dimethicon | - | 1,00 |
| Glycerin | 3,00 | 7,50 |
| Methylparaben | - | 0,20 |
| Phenoxyethanol | 0,10 | 0,20 |
| Propylparaben | 0,10 | 0,10 |
| Na-disulfit | 0,20 | - |
| Carbomer | 0,10 | 0,10 |
| Trinatrium EDTA | - | 1,00 |
| Ammonium Acryloyldimethyltaurat/VP Crosspolymer | 0,25 | - |
| Tapiokastärke | - | 2,50 |
| Talkum | 2,00 | - |
| Creatin | - | 0,75 |
| Creatinin | - | 0,10 |
| Zingeron | 0,05 | 1,00 |
| NaOH | q.s. | q.s. |
| Parfum | q.s. | q.s. |
| Ethylhexyl Methoxycinnamate | - | 2,00 |
| Butyl Methoxydibenzoylmethane | - | 2,00 |
| Phenylbenzimidazolsulfonsäure | - | 1,00 |
| Wasser | ad 100 | ad 100 |

## Patentansprüche

1. Verwendung von Zingeron zur Behandlung und Prophylaxe der Symptome der intrinsischen und/oder extrinsischen Hautalterung.

## Claims

1. Use of zingerone for the treatment and prophylaxis of the symptoms of intrinsic and/or extrinsic skin ageing.

## Revendications

1. Utilisation de Zingeron pour le traitement et la prophylaxie des symptômes du vieillissement intrinsèque et/ou extrinsèque de la peau.
